Europäisches Patentamt

European Patent Office

Office européen des brevets

(19)

(11) Publication number: **0 110 173**
**B1**

(12) # EUROPEAN PATENT SPECIFICATION

(45) Date of publication of patent specification: 04.03.87

(21) Application number: 83110873.3

(22) Date of filing: 31.10.83

(51) Int. Cl.⁴: **C 12 Q 1/26,** C 12 Q 1/54,
G 01 N 33/52

(54) **Test device and method for in a colored aqueous fluid by diffuse reflectance detecting a soluble analyte measurement.**

(30) Priority: 01.11.82 US 438399

(43) Date of publication of application:
13.06.84 Bulletin 84/24

(45) Publication of the grant of the patent:
04.03.87 Bulletin 87/10

(84) Designated Contracting States:
AT BE CH DE FR GB IT LI LU NL SE

(56) References cited:
DE-A-2 264 438
FR-A-2 378 277
US-A-4 125 372

ANALYTICAL CHEMISTRY, vol. 55, no. 4, April
1983, pages 498A-514A, Columbus, Ohio, USA
B. WALTER: "Dry reagent chemistries"

(73) Proprietor: Lifescan, Inc.
1025 Terra Bella Avenue
Mountain View California 94043 (US)

(72) Inventor: Phillips, Roger C.
845 Richardson Ct.
Palo Alto, CA. 94303 (US)
Inventor: Thomson, Richard P.
3133 Stevens Court
San Jose, CA. 95148 (US)
Inventor: Underwood, Raymond D.
13020 La Vista Drive
Saratoga, CA. 95070 (US)
Inventor: McCarraugh, Geoffrey V.
291 Hacienda Drive
Scotts Valley, CA. 95066 (US)

(74) Representative: Behrens, Dieter, Dr.-Ing. et al
Patentanwälte WUESTHOFF-V. PECHMANN-
BEHRENS-GOETZ Schweigerstrasse 2
D-8000 München 90 (DE)

**Description**

Field of the invention

The present invention relates to a test device and method for the colorimetric determination of chemical and biochemical components (analytes) in colored aqueous fluids, particularly colored biological fluids. In one preferred embodiment it concerns a test device and method for colorimetrically measuring the concentration of glucose in whole blood.

Background of the invention

The quantification of chemical and biochemical components in colored aqueous fluids and, in particular, colored biological fluids such as whole blood and urine and biological fluid derivatives such as blood serum and blood plasma is of every-increasing importance. It finds application in connection with analysis of these fluids and with medical diagnosis and treatment and in the quantification of exposure to therapeutic drugs, intoxicants, hazardous chemicals and the like. In some instances, the amounts of materials being determined are either so miniscule—in the range of a microgram or less per deciliter or so difficult to precisely determine that the apparatus employed is complicated and useful only to skilled laboratory personnel. In this case the results are generally not available for some hours or days after sampling. In other instances, there is often an emphasis on the ability of lay operators to perform the test routinely, quickly and reproducibly outside a laboratory setting with rapid or immediate information display.

A variety of dry reagent chemistries have been proposed for carrying out a range of analyses on test strips. See, for example, the article "Dry Reagent Chemistries is Clinical Analysis" by B. Walter, appearing at Analytical Chemistry Vol. 55, No. 4, April, 1983 at pages 498—514.

One common routine medical test is the measurement of blood glucose levels by diabetics. Current teaching counsels diabetic patients to measure their blood glucose level anywhere from two to seven times a day depending on the nature and severity of their individual cases. Based on the observed pattern in the measured glucose levels the patient and physician together make adjustments in diet, exercise and insulin intake to better manage the disease. Clearly, this information should be read out to the patient immediately.

Currently a method widely used in the United States employs a test article of the type described in U.S. Patent 3,298,789 issued January 17, 1967 to Mast. In this method a sample of fresh, whole blood (typically 20—40 µl) is placed on an ethylcellulose-coated reagent pad containing an enzyme system having glucose oxidase and peroxidase activity. The enzyme system reacts with glucose and releases hydrogen peroxide. The pad also contains an indicator which reacts with the hydrogen peroxide in the presence of peroxidase to give a color proportional in intensity to the sample's glucose level.

The other most popular blood glucose test method employs similar chemistry but in place of the ethylcellulose-coated pad employs a water resistant film through which the enzymes and indicator are dispersed. This type of system is disclosed in United States Patent 3,630,957 issued December 28, 1971 to Rey et al.

In both cases the sample is allowed to remain in contact with the reagent pad for a specified time (typically one minute). Then, in the first case the blood sample is washed off with a stream of water while in the second case it is wiped off the film. The reagent pad or film is then blotted dry and evaluated. The evaluation is made either by comparing color generated with a color chart or by placing the pad or film it in a diffuse reflectance instrument to give a color intensity value.

While the above methods have been used in glucose monitoring for years, they do have certain limitations. The sample size required is rather large for a finger stick test and is difficult to achieve for some people whose capillary blood does not express readily.

In addition, these methods share a limitation with other simple lay operator colorimetric determinations in that their result is based on an absolute color reading which is in turn related to the absolute extent of reaction between the sample and the test reagents. The fact that the sample must be washed or wiped off the reagent pad after the timed reaction interval requires that the user be ready at the end of the timed interval and apply the wash stream or wipe at the required time. The fact that the reaction is stopped by removing the sample leads to some uncertainty in the result especially in the hands of the home user. Overwashing can give low results and underwashing can give high results.

The presence of red blood cells or other colored components often interferes with the measurements of these absolute values thereby calling for exclusion of red blood cells in these two prior methods as they are most widely practiced. In the device of patent 3,298,789 an ethylcellulose membrane prevents red blood cells from entering the reagent pad. Similarly, the water-resistant film of patent 3,630,957 prevents red blood cells from entering. In both cases the rinse or wipe is required to remove these potentially interfering red blood cells prior to measurement. In these devices the reagent pad or film is relatively thick—i.e., at least 0.3 mm and often 0.5 mm or more. This same interference would be likely to occur with other colored fluids, as well.

The invention, as defined by the appended claims, overcomes these limitations and provides a test device comprising a reagent containing flat sample pad on a backing and a method for rapid readout routine but accurate colorimeter determination of analytes. The test device accomplishes this by being

2

made up of a thin layer of hydrophilic substrate containing color-forming reagent attached to a reflective backing. The substrate is light transmissive and serves as the sample-holding color-forming zone. Color formation in the color-forming zone is determined on a color change—i.e. a rate of change or a color difference rather than on the absolute color value achieved and from this color change, the glucose level is calculated. The invention provides an improved blood glucose determination in whole blood without the need to eliminate red blood cells, but can be employed to measure other analytes in other colored aqueous solutions, particularly colored biologic solutions.

The hydrophilic pad contains the reagent which reacts with the glucose to yield a compound that absorbs at the wavelength of measurement, the pad being substantially translucent at the wavelength of said measurement and being affixed to a diffuse reflective backing.

In a preferred embodiment for detecting glucose in whole blood the hydrophilic cellulose pad has a 10 to 50 mm$^2$ area through which at least about 35% of incident 600 nanometer or greater wavelength light can pass and is impregnated with a glucose oxidase, a substance having peroxidase activity and a substance which reacts with hydrogen peroxide to form a compound that absorbs in the 600 nanometer or greater range and in that the pad is affixed to the diffuse reflective backing such that at least about 35% of incident light is diffusely reflected.

The method in accordance with the invention which comprises bringing a sample of the fluid, especially whole blood, into contact with the sample pad of the test device according to the invention comprises projecting a light beam of an appropriate wavelength through the sample pad and diffusely reflecting said beam off the reflective backing, observing by diffuse reflectance measurement the light intensity change at the wavelength of measurement, and relating the intensity change to the concentration of analyte.

A preferred embodiment for determining the concentration of glucose in whole blood is characterized by bringing a 5—20 microliter sample of whole blood in contact with the test pad of the above-mentioned test device, projecting a 635 nanometer light beam through said pad and observing by diffuse reflectance measurement the intensity change of the beam diffusively reflected off of the diffusive backing at 635 nanometer and relating the intensity change to the concentration of glucose.

The invention also provides a system for determining the concentration of glucose-containing whole blood comprising a test strip including a diffuse reflective backing to which is affixed a sample pad into which the whole blood is applied, said pad being flat, capable of transmitting 600 nanometer or greater wavelength light, consisting of hydrophilic cellulose, 0.03 to 0.40 mm thick and impregnated with an indicator comprising glucose oxidase, a peroxidase and a compound capable of generating color by reaction with hydrogen peroxide, means for determining the generated color by diffuse reflectance measurements, means for reading the diffuse reflectance measurement at at least two predetermined set time intervals after the whole blood has been applied into the sample pad, means for converting the difference in diffuse reflectance measurement to read as a concentration of glucose in the whole blood and means for displaying the glucose concentration so obtained.

Two embodiments of a test device according to the invention are described in the drawings:

Figure 1 is a perspective view of one embodiment of the test device.

Figure 2 is a perspective view of an alternative embodiment of the test device.

Figure 3 is a block diagram schematic of an apparatus which can be employed in the practice of the invention.

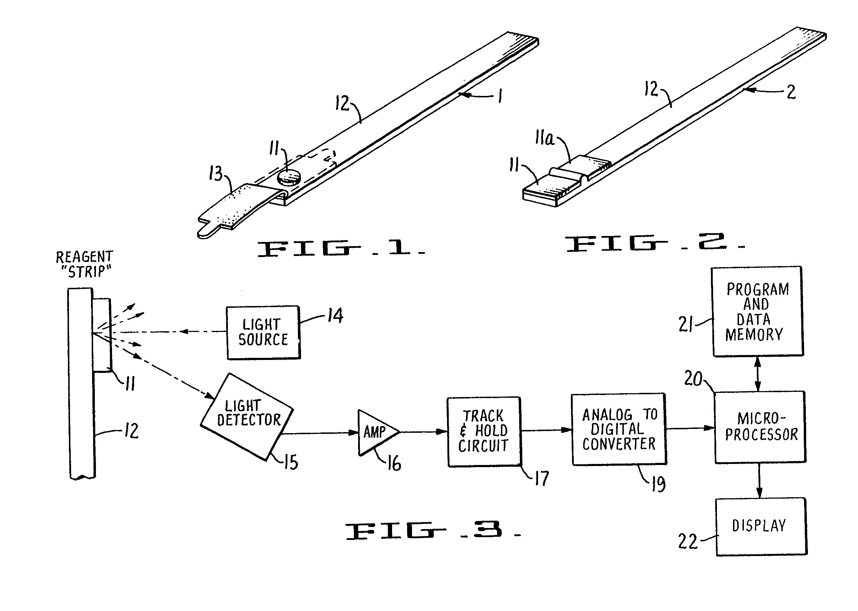

As shown in Figure 1 the test device of this invention has a thin translucent hydrophilic substrate pad 11 positioned on a diffuse reflective backing 12. Optionally, pad 11 is covered by tap 13, which is removed in use but is removably sealed around pad 11 prior to use. Pad 13 can be made of foil, waterproofed paper, or water and light impermeable protective plastic such as poly(ethylene) or poly(vinylchloride) or the like and is provided to protect pad 11 from degradation prior to its use in testing. Pad 11 contains a reagent system. In use, the colored aqueous sample being analyzed is applied to pad 11 and any analyte present reacts with the reagent system to give a colored compound. When the test device is placed in a diffuse reflectance spectrophotometer, incident light passes through the hydrophilic layer containing the colored sample and reagent, is reflected from the backing and is emitted from the surface of the device as diffuse reflected light. This diffuse light can be collected and measured such as by the detector of the diffuse reflectance spectrophotometer. Its amount will be an inverse function of the concentration of analyte in the sample. Substrate 11 may be of any water-insoluble hydrophilic material including natural materials like cellulose, paper, cotton, silk, and cross-linked gelatin and synthetic materials such as cross-linked hydroxymethylacrylates and acrylates, cellulose acetate, cellulose nitrate, cross-linked poly(vinyl alcohol), poly(vinylamine), poly(vinylsulfonate) copolymers, poly(styrene sulfonate) and copolymers containing styrene sulfonate units, poly(vinyl acetate), poly(maleic anhydride), poly(vinyl 2-methoxyethyl ether), poly(4-vinyl phthalic acid), poly(N-vinylmorpholinone), poly(N-vinylpyrrolidone), poly(methacrylic acid), poly(acrylamide), poly(methacrylamide), poly(ethylene oxide) and the like. The hydrophilic material may be a gel layer, porous or fibrous, or the like so long as in its form it is light transmissive. Cellulose and cellulose derivatives are readily available, work fine and thus are preferred hydrophilic substrates. The hydrophilic substrate should be thin enough to be substantially translucent, that is to permit substantial passage of light. Preferably, the pad permits at least 25% of the incident light applied to it to reach the reflective diffuse reflectance. More preferably, at least 35% of the incident light applied is reflected and emitted as diffuse reflectance. This is accomplished for a cellulose fiber substrate if it is less than about 0.4 mm thick. If it is too thin, however, it is difficult for it to contain enough sample to develop substantial color. In general this occurs if the substrate is less than about 0.01 mm. A preferred thickness for cellulose fibers is from about 0.03 mm to about 0.4 mm, especially from about 0.05 mm to 0.3 mm, with a more preferred thickness being from 0.10 to 0.20 mm. A liquid sample is applied to pad 11. Substrate pad 11 is sized to be smaller in volume than the sample which is applied to it. If it is larger or similar in volume to the sample, there are problems with the sample pad "chromatographing" the sample or "spreading" the sample and giving an uneven result. Generally, when a sample of fresh blood is the sample being tested the test pad should be on the order to 10 mm² to 100 mm² in area, especially 10 mm² to 50 mm² in area, and preferably such that 5—10 microliters of sample will more than saturate it. The sample pad contains chemical reagents which will react with the analyte and produce a colored compound. These will be described separately, below. The sample pad is attached to backing 12. Backing 12 is reflective. In a diffuse reflective system, as is employed herein, a perfectly matte white surface would be ideal. Such a surface can be achieved with a white opaque plastic or any other material which provides an impermeable opaque backing. Such backings can include, for example titanium dioxide—whitened poly(styrene), poly(vinylchloride) or poly(ethylene) or the like; unpolished metals such as aluminum, zinc or the like; white painted metals, wood, or the like; hydrophobic paper products such as water-repellant-treated board stock, heavy papers or the like. Opaque plastics offer the additional features of inexpensiveness, strength and rigidity and thus permit easy insertion in test apparatus and are preferred.

In an alternative embodiment, the test device can include two adjacent but discontinuous hydrophilic sample pads—one pad contains the test reagents, the other is a "blank" and does not contain all the reagents necessary to generate the colored compound. Such a test device is shown in Fig. 2 as device 2 wherein 11 and 11a are the two sample pads. The two pads should be close enough together to permit essentially simultaneous sample addition but separate so as to prevent migration and contamination between the test pad and the "blank" pad.

The hydrophilic layer may be held against the reflective backing by a holder or clamp, however, in the preferred method it is bonded to the backing. The bonding can be done with any adhesive that cures to a clear or white state or by a thermal method in which the backing surface is melted enough to entrap some of the material used for the hydrophilic layer, or by microwave or ultrasonic bonding methods which likewise fuse the hydrophilic sample pads to the backing. It is important that the bonding be such as to not itself interfere substantially with the diffuse reflectance measurements.

The chemical reagents

This invention is not to be construed as limited by the precise chemical reagents employed. In general terms, it is anticipated that any reagents may be employed that are capable of reacting with the analyte in the sample to produce a compound that is characteristically absorptive at a wavelength other than a wavelength at which the colored sample itself substantially absorbs. Nonlimiting examples of analyses and typical reagents include the following materials shown in Table I.

4

# 0 110 173

TABLE I

| Analyte and sample type | Reagents |
|---|---|
| Glucose in blood, serum urine or other biological fluids, wine, fruit juices or other colored aqueous fluids. | Glucose oxidase, peroxidase and an oxygen acceptor<br><br>Oxygen acceptors include:<br>O-dianisidine (1)<br>O-toluidine<br>O-tolidine (1)<br>Benzidine (1)<br>2,2'-Azinodi-(3-ethylbenzthiazoline sulphonic acid-(6)) (1)<br>3-Methyl-2-benzothiazolinone hydrazone plus N,N-dimethylaniline (1)<br>Phenol plus 4-aminophenazone (1)<br>Sulfonated 2,4-dichlorophenol plus 4-aminophenazone (2)<br>3-Methyl-2-benzothiazolinone hydrazone plus 3-(dimethyl amino)benzoic acid (3)<br>2-Methoxy-4-allyl phenol (4)<br>4-Aminoantipyrinedimethyl-aniline (5) |
| Galactose in aqueous | Galactose oxidase, catalase and an oxygen acceptor as noted above. |
| Alcohol in whole blood, plasma, blood serum, urine, fruit, juice, wine, beer, etc. | Alcohol oxidase enzyme available commercially from Phillips Petroleum Company (the enzyme system and its use are described in *Biochim Biophys Acta, 151,* 303, and *Chemical Abstracts, 94,* 135580a, peroxidase, and an oxygen acceptor as set forth above. |
| Ketone in whole blood, plasma, serum, wine, etc. | Nitroprusside, and acetoacetic acid as set forth in German OLS 2,158,125 May 1973. |
| Cholesterol in serum, whole blood, etc. | Cholesterol oxidase, cholesterol esterase, peroxidase and an oxygen acceptor as set forth above. This chemistry is described at *Clin Chem* 24/12 2161—2165 (1978). |

In addition, the following analytes are known to have specific oxidase enzymes which will react and generate $H_2O_2$. By incorporating a peroxidase and an oxygen acceptor as set forth above, a suitable test would result.

| | |
|---|---|
| Glycollate | D-Aspartate |
| L-Malate | A L-amino acid |
| Aryl alcohol | A D-amino acid |
| L-2-hydroxy acid | Glutamate |
| Choline | Tyramine |
| Pyruvate | Putrescine |
| Oxalate | Sarcosine |
| Glyoxalate | |

(1) As reported *Clinical Chemistry*, Richterich and Columbo, P. 367 and references cited therein.
(2) *Analyst, 97,* (1972) 142—5.
(3) *Anal. Biochem. 105,* (1980) 389—397.
(4) *Anal. Biochem. 79,* (1977) 597—601.
(5) *Clinica Chemica Acta, 75,* (1977) 387—391.

5

The analysis method

The analysis method of this invention relies on a change in absorbance, as measured by diffuse reflectance, which is dependent upon the amount of analyte present. This change can be determined in either of two modes—either by comparison of the test sample with a blank preferably after a set minimum time interval or by measuring the change in the absorbance of the test sample between two or more points in time. The first mode is especially useful when the reaction to form the colored compound is rapid or essentially instantaneous. The second mode is most useful with somewhat slower reactions so that over a period of time a rate of change can be detected and based on this rate, a concentration of analyte determined. The latter method, being based on the rate of change may be especially advantageous as it is generally less sensitive to sample to sample variation.

In practice, a test could be carried out as follows: First a sample of aqueous fluid containing an analyte is obtained. An excess over saturation (i.e. about 5 to 10 microliters) of this fluid is applied to the hydrophilic pad or pads of the test device and as simultaneously as possible a timer is started. The pad is saturated and excess fluid is removed such as by light blotting and the test device is placed in an instrument for reading absorbance—i.e. color intensity. Color intensity is measured at certain points in time after application of the sample. From these measurements of color intensity a rate of color development can be calibrated in terms of analyte level. Thus a suitable instrument, such as a diffuse reflectance spectrophotometer with appropriate software can be made to automatically read color intensity at certain points in time, calculate rate of color change and using calibration factors, output the level of analyte in the aqueous fluid. Such a device is schematically shown in Fig. 3 wherein a test device of the invention comprising backing 12 to which reagent pad 11 is affixed is shown. Light source 14, for example a high intensity light emitting diode (LED) projects a beam of light onto the test device. A substantial portion (at least 25% and preferably at least 35% and more preferably at least 50% of this light reflects off of backing 12 and is diffusively reflected where it is detected by light detector 15, a phototransistor that produces an output current proportional to the light it receives. Light source 14 and/or detector 15 can be adapted to generate or respond to a particular wavelength light if desired. The output of detector 15 is passed to amplifier 16, a linear integrated circuit which converts the phototransistor current to a voltage. The output of amplifier 16 is fed to track and hold circuit 17. This is a combination linear/digital integrated circuit which tracks or follows the analog voltage from amplifier 16 and upon command from microprocessor 20, locks or holds the voltage at its level at that time. Analog-to-digital converter 19, takes the analog voltage from track and hold circuit 17 and converts it to a twelve-bit binary digital number upon command of microprocessor 20. Microprocessor 20 is a digital integrated circuit. It serves the following function: 1) It serves as a timer for the entire system; 2) It reads the output of analog/digital converter 19; 3) It, together with program and data memory 21 stores data corresponding to the reflectance measured at specified time intervals; 4) It calculates analyte levels from the stored reflectances; and 5) It outputs analyte concentration data to display 22. Memory 21 is a digital integrated circuit which stores data and the microprocessor operating program. Display 22 can take various hard copy and soft copy forms. Usually, it is a visual display, such as a liquid crystal or LED display but it can also be a tape printer or the like. Display 22 also includes a start-stop switch and can also provide an audible or visible time output to indicate times for applying samples, starting timers, etc.

When compared to prior methods for determining glucose in blood there are several advantages. First the amount of sample required to saturate the thin hydrophilic layer is small (typically 5—10 microliters). Second, operator time required is only that necessary to apply the sample to the thin hydrophilic layer, blot off excess sample and insert it into the instrument (typically 4—7 seconds). Third, it can base its determinations on rate measurements. Rate methods are inherently more accurate than endpoint measurements. Fourth, this method functions with whole blood and does not require any separation or utilization of red cell—free samples and likewise can be used with other deeply-colored samples.

The invention will be further described by the following examples. These are provided to illustrate the invention and are not to be construed as limiting its scope.

Example I
Determination of glucose in whole blood

An indicator solution was made by mixing 2.0 ml ethanol, 40 mg dimethylaminobenzoic acid (DMAB), 2.5 ml $H_2O$, 1.0 ml $Na_2HPO_4$, 0.40M, 1.0 ml gluconic acid potassium salt 10% wt. in water, 15 mg 3-methylbenzothiazolinone hydrazone (MBTH), 100 mg dimethylamino naphthalene sulfonic acid (DMANS), and 2.5 ml acacia 10% wt. in water. The solution was stirred until all components were dissolved.

An enzyme solution was made by mixing 1.6 ml $H_2O$, 0.40 ml glucose oxidase of 1200 IU/ml, and 5 mg horseradish peroxidase as a lyophilized powder.

The indicator and enzyme solutions were mixed and the pH was adjusted with 1.0M NaOH to pH 6.2.

A 10 cm square of 0.15 cm thick 99+% alpha cellulose (Whatman 541 filter paper) was saturated with the combined reagent solution and dried in a circulating air oven for 10 minutes at 56°C.

The reagent paper thus formed was cut into 5 mm×10 cm strips and fixed to a 15 mil thick white polystyrene backing 4 cm×10 cm in size using a 3M transfer adhesive to give a card with reagent paper

6

along one edge. The card was then cut into reagent strips 5 mm×4 cm in size with a 5 mm×5 mm reagent pad on one end.

A sample of whole blood (5—10 µl) was applied to a reagent strip and excess sample blotted off. The strip was then placed in a diffuse reflectance spectrophotometer and color intensity readings were taken at a wavelength of 635 nanometers, 7 seconds and 27 seconds after application of sample. A value for the rate of color development, called a $DA_{20}$ value, was then determined as follows:

$$DA_{20} = \log\left(\frac{R_7}{R_{27}}\right)$$

wherein $R_7$ is the reflectance value at 7 seconds and $R_{27}$ is the value at 27 seconds. The $DA_{20}$ value was then compared with $DA_{20}$ values determined with standard solutions of glucose concentration in the test sample. This was carried out both manually and under control of a microprocessor as shown in Fig. 3.

Example II
Determination of sugar and alcohol levels during wine production

Grapes are harvested for wine production at optimum sugar levels. This sugar is predominantly glucose ("grape sugar") at 15 to 25%. During fermentation, some or all of the glucose is converted to alcohol. Test strips are prepared as shown in Example I. The strip is calibrated using grape juices adjusted to provide known concentrations of glucose. The resulting calibrated strips may then be used as generally set forth in Example I with samples of grape juice in the field, to quickly determine optimal harvest times, and with samples of wine must to determine fermentation progress.

The build up of alcohol can be measured directly by this invention, as well. An alcohol test strip can be prepared using the general method of Example I with the change that an alcohol oxidase enzyme as reported in *Biochim Biophys Acta, 151*, 303 is used in place of the glucose oxidase. This strip is calibrated with solutions of known ethanol content and, based on this calibration, used in the method of Example I to rapidly determine ethanol levels in wine fermentation musts and in finished wine products.

Example III
The alcohol test strip of Example II is used to determine levels of ethanol in human blood, human blood plasma, and human blood serum.

Example IV
Examples I and II are repeated with the change that a two pad test device of Fig. 2 is employed and the levels of glucose and ethanol are determined based on the change in the sample pad relative to the blank pad over a set time interval.

**Claims**

1. Test device for detecting a soluble analyte, particularly glucose or ethanol, in a colored aqueous fluid, especially whole blood, blood serum and blood plasma, fruit based fluid or biological solution, by diffuse reflectance measurement, comprising a reagent containing flat sample pad on a backing characterized by a hydrophilic pad (11) containing the reagent which reacts with the analyte to yield a compound that absorbs at the wavelength of measurement, said pad (11) being substantially translucent at the wavelength of said measurement and being affixed to a diffuse reflective backing (12).

2. Test device of claim 1 characterized in that the reagent comprises an oxidase for the analyte, a substance having peroxidase activity and a substance which will react with hydrogen peroxide to form a colored compound.

3. Test device according to claim 1 or 2 characterized in that said pad (11) comprises cellulose.

4. Test device according to claim 3 characterized in that said pad (11) has a thickness of 0.03 to 0.4 mm.

5. Test device according to claim 3 for detecting glucose or ethanol of whole blood, characterized in that said pad (11) has a thickness of 0.05 to 0.3 mm.

6. Test device according to claim 4 for detecting glucose in whole blood characterized in that the hydrophilic cellulose pad (11) has a 10 to 50 mm² area through which at least about 35% of incident 600 nanometer or greater wavelength light can pass and is impregnated with a glucose oxidase, a substance having peroxidase activity and a substance which reacts with hydrogen peroxide to form a compound that absorbs in the 600 or greater nanometer range and in that the pad (11) is affixed to the diffuse reflective backing (12) such that at least about 35% of incident light is diffusely reflected.

7. Method for determining the concentration for an analyte, particularly glucose or ethanol, in a colored aqueous fluid, especially whole blood, blood serum, blood plasma, fruit based fluid or biological solution, which comprises bringing a sample of the fluid into contact with the sample pad of the test device according to any of claims 1 to 6 and performing a colorimetric determination, characterized by projecting a light beam of an appropriate wavelength through the sample pad and diffusely reflecting said beam off the

7

# 0 110 173

reflective backing, observing by diffuse reflectance measurement the light intensity change at the wavelength of measurement, and relating the intensity change to the concentration of analyte.

8. Method according to claim 7 for determining the concentration of glucose in whole blood characterized by bringing a 5—20 microliter sample of whole blood in contact with the test pad of the test device of claim 6, projecting a 635 nanometer light beam through said pad and observing by diffuse reflectance measurement the intensity change of the beam diffusively reflected off of the diffusive backing at 635 nanometer and relating the intensity change to the concentration of glucose.

9. Method according to claim 7 or 8 characterized in that the intensity change is determined by comparing a blank with the test pad.

10. Method according to claim 7 or 8 characterized in that the intensity change is determined by comparing the intensity at two times after the sample is applied to the sample pad.

11. System for determining the concentration of glucose-containing whole blood comprising a test strip including a diffuse reflective backing (12) to which is affixed a sample pad (11) into which the whole blood is applied, said pad being flat, capable of transmitting 600 nanometer or greater wavelength light, consisting of hydrophilic cellulose, 0.03 to 0.40 mm thick and impregnated with an indicator comprising glucose oxidase, a peroxidase and a compound capable of generating color by reaction with hydrogen peroxide, means (15) for determining the generated color by diffuse reflectance measurements, means (20) for reading the diffuse reflectance measurement at at least two predetermined set time intervals after the whole blood has been applied into the sample pad (11), means (20, 21) for converting the difference in diffuse reflectance measurement to read as a concentration of glucose in the whole blood and means (22) for displaying the glucose concentration so obtained.

**Patentansprüche**

1. Testvorrichtung zum Bestimmen eines löslichen Analyten, insbesondere Glucose oder Ethanol, in einer farbigen wässrigen Flüssigkeit, insbesondere in Gesamtblut, Blutserum und Blutplasma, Fruchtsaft oder bilogischer Lösung, durch Messen des diffusen Reflexionsvermögens, mit einem flachen Probenkissen zur Aufnahme eines Reagens auf einer Unterlage, gekennzeichnet durch ein hydrophiles Kissen (11), welches das Reagens enthält, das mit dem Analyten unter Bildung einer Verbindung reagiert, die bei der Meßwellenlänge absorbiert, wobei das Kissen (11) für die Wellenlänge der Messung im wesentlichen durchscheinend und auf einer diffus reflektierenden Unterlage (12) befestigt ist.

2. Testvorrichtung nach Anspruch 1, dadurch gekennzeichnet, daß das Reagens eine Oxidase für den Analyten, einen Stoff mit Peroxidase-Aktivität und einen Stoff, der mit Wasserstoffperoxid unter Bildung einer farbigen Verbindung reagiert, umfaßt.

3. Testvorrichtung nach Anspruch 1 oder 2, dadurch gekennzeichnet, daß das Kissen (11) Zellulose umfaßt.

4. Testvorrichtung nach Anspruch 3, dadurch gekennzeichnet, daß das Kissen (11) zwischen 0,03 und 0,4 mm dick ist.

5. Testvorrichtung nach Anspruch 3 zum Bestimmen von Glucose oder Ethanol in Gesamtblut, dadurch gekennzeichnet, daß das Kissen (11) zwischen 0,05 und 0,3 mm dick ist.

6. Testvorrichtung nach Anspruch 4 zum Bestimmen von Glucose in Gesamtblut, dadurch gekennzeichnet, daß das Kissen (11) aus hydrophiler Zellulose eine Fläche von 10 bis 50 mm² hat, durch die wenigstens etwa 35% von einfallendem Licht mit der Wellenlänge 600 Nanometer oder größer hindurchgehen kann, und mit einer Glucose-Oxidase, einem Stoff mit Peroxidase-Aktivität und einem Stoff imprägniert ist, der mit Wasserstoffperoxid unter Bildung einer Verbindung reagiert, die im Bereich 600 Nanometer oder größer absorbiert, und daß das Kissen (11) an der diffus reflektierenden Unterlage (12) so befestigt ist, daß wenigstens etwa 35% des einfallenden Lichtes diffus reflektiert werden.

7. Verfahren zur Konzentrationsbestimmung für einen Analyten, insbesondere Glucose oder Ethanol, in einer farbigen wässrigen Flüssigkeit, insbesondere in Gesamtblut, Blutserum, Blutplasma, Fruchtsaft oder biologischer Lösung, bei dem eine Probe der Flüssigkeit mit dem Probenkissen der Testvorrichtung nach einem der Ansprüche 1 bis 6 in Kontakt gebracht und eine kolorimetrische Bestimmung durchgeführt wird, dadurch gekennzeichnet, daß ein Lichtstrahl von zweckdienlicher Wellenlänge durch das Probenkissen hindurchgeschickt und an der reflektierenden Unterlage diffus reflektiert wird, durch Messen des diffusen Reflexionsvermögens die Lichtintensitätsänderung bei der Meßwellenlänge beobachtet wird, und die Intensitätsänderung zur Analytkonzentration in Beziehung gesetzt wird.

8. Verfahren nach Anspruch 7 zum Bestimmen der Glucosekonzentration in Gesamtblut, dadurch gekennzeichnet, daß eine Gesamtblutprobe von 5 bis 20 Mikroliter mit dem Testkissen der Testvorrichtung nach Anspruch 6 in Kontakt gebracht wird, ein Lichtstrahl von 635 Nanometer durch das Kissen hindurchgeschickt wird, und durch Messen des diffusen Reflexionsvermögens die Intensitätsänderung bei 635 Nanometer des an der streuenden Unterlage diffus reflektierten Lichtstrahls beobachtet wird, und die Intensitätsänderung zur Glucosekonzentration in Beziehung gesetzt wird.

9. Verfahren nach Anspruch 7 oder 8, dadurch gekennzeichnet, daß die Intensitätsänderung durch Vergleichen einer Leerprobe mit dem Testkissen ermittelt wird.

10. Verfahren nach Anspruch 7 oder 8, dadurch gekennzeichnet, daß die Intensitätsänderung durch zweimaliges Vergleichen der Intensität nach dem Auftragen der Probe auf das Probekissen ermittelt wird.

8

## 0 110 173

11. System zum Bestimmen der Konzentration von Glucose enthaltendem Gesamtblut, mit einem Teststreifen mit einer diffus reflektierenden Unterlage (12), an der ein Probekissen (11) befestigt ist, in das das Gesamtblut aufgegeben wird, wobei das Kissen flach ist, Licht mit einer Wellenlänge von 600 Nanometer oder größer durchläßt, aus hydrophiler Zellulose besteht, 0,03 bis 0,40 mm dick und mit einem Indikator imprägniert ist, der Glucose-Oxidase, eine Peroxidase und eine Verbindung umfaßt, die durch Reaktion mit Wasserstoffperoxid eine Farbe zu erzeugen vermag, einer Einrichtung (15) zum Bestimmen der erzeugten Farbe durch Messen des diffusen Reflexionsvermögens, einer Einrichtung (20) zum Ablesen der Meßwerte für das diffuse Reflexionsvermögen in wenigstens zwei im voraus festgelegten Zeitabständen nach Aufgeben des Gesamtblutes in das Probenkissen (11), Einrichtungen (20, 21) zum Umwandeln der Differenz der Meßwerte für das diffuse Reflexionsvermögen in eine Anzeige der Glucosekonzentration im Gesamtblut, und einer Einrichtung (22) zum Anzeigen der so ermittelten Glucosekonzentration.

## Revendications

1. Trousse d'essai pour la détermination d'un analyte soluble, plus particulièrement de la glucose ou de l'éthanol, dans un fluide aqueux coloré, spécialement dans du sang complet, du sérum et du plasme de sang, du jus de fruit ou dans des solutions biologiques, en mesurant la réflectance diffuse, comprenant un coussin plat à échantillons, disposé sur un élément de base et contenant un réactif, caractérisée par un coussin hydrophile (11), contenant le réactif qui réagit avec l'analyte en formant un composé absorbant à la longueur d'onde utilisée pour la mesure, ledit coussin (11) étant, en général, translucent à la longueur d'onde de cette mesure, et étant attaché à un élément de base (12) capable de renvoyer en réflexion diffuse.

2. Trousse d'essai selon la revendication 1, caractérisée en ce que le réactif comprend une oxydase pour l'analyte, une substance possédant une activité de peroxydase, et une substance qui réagira avec le peroxyde d'hydrogène en formant un composé coloré.

3. Trousse d'essai selon l'une quelconque des revendications 1 et 2, caractérisée en ce que ledit coussin (11) comprend de la cellulose.

4. Trousse d'essai selon la revendication 3, caractérisée en ce que ledit coussin (11) à une épaisseur comprise entre 0,03 et 0,4 mm.

5. Trousse d'essai selon la revendication 3, pour la détermination de la glucose ou de l'éthanol de sang complet, caractérisée en ce que ledit coussin (11) a une épaisseur comprise entre 0,05 et 0,3 mm.

6. Trousse d'essai selon la revendication 4, pour la détermination de la glucose dans du sang complet, caractérisée en ce que le coussin (11) en cellulose hydrophile présente une superficie comprise entre 10 et 50 mm², à travers laquelle au moins environ 35 pour cent d'une lumière incidente de longueur d'onde de 600 nanomètres ou davantage peuvent être transmis, et est imprégné d'une glucose-oxydase, d'une substance possédant une activité de peroxydase, et d'une substance qui réagit avec le peroxyde d'hydrogène en formant un composé qui absorbe dans la région de 600 nanomètres ou davantage, et en ce que le coussin (11) est attaché à l'élément de base (12), capable d'une réflexion diffuse, de manière qu'au moins 35 pour cent, environ, de la lumière incidente sont renvoyés en réflexion diffuse.

7. Procédé de détermination de la concentration pour un analyte, plus particulièrement pour de la glucose ou de l'éthanol, dans un fluide aqueux coloré, spécialement dans du sang complet, du sérum ou du plasme de sang, du jus de fruit ou dans des solutions biologiques, procédé dans lequel on met un échantillon, pris dans le fluide, en contact avec le coussin à échantillons de la trousse d'essai selon l'une quelconque des revendications 1 à 6, et on effectue une détermination colorimétrique, caractérisé en ce que l'on fait passer un faisceau de lumière d'une longueur d'onde appropriée, à travers le coussin à échantillons et on le fait renvoyer en réflexion diffuse par l'élément de base réfléchissant; puis l'on observe, en mesurant la réflectance diffuse, le changement de l'intensité de la lumière à la longueur d'onde utilisée pour la mesure; et l'on établit une relation entre le changement d'intensité et la concentration de l'analyte.

8. Procédé selon la revendication 7, pour la détermination de la concentration de glucose dans du sang complet, caractérisé en ce que l'on met un échantillon, de 5 à 20 microlitres, de sang complet en contact avec le coussin d'essai de la trousse d'essai selon la revendication 6; on fait passer un faisceau de lumière de 635 nanomètres à travers ledit coussin; puis l'on observe, en mesurant la réflectance diffuse, le changement d'intensité, à 635 nanomètres, du faisceau qui est renvoyé en réflexion diffuse par l'élément de base réfléchissant; et l'on établit une relation entre le changement d'intensité et la concentration de glucose.

9. Procédé selon l'une quelconque des revendications 7 et 8, caractérisé en ce que le changement d'intensité est déterminé en comparant un échantillon à blanc avec le coussin d'essai.

10. Procédé selon l'une quelconque des revendications 7 et 8, caractérisé en ce que le changement d'intensité est déterminé en comparant l'intensité à deux moments après l'application de l'échantillon au coussin à échantillons.

11. Système pour la détermination de la concentration d'un sang complet contenant de la glucose, comprenant une bande de test ayant un élément de base (12), capable d'une réflexion diffuse, auquel est attaché un coussin à échantillons (11), dans lequel on applique le sang complet, ce coussin étant plat, capable de transmettre de la lumière d'une longueur d'onde de 600 nanomètres ou davantage, consistant

9

en cellulose hydrophile, ayant une épaisseur comprise entre 0,03 et 0,40 mm, et étant imprégné d'un indicateur comprenant de la glucose-oxydase, une peroxydase et un composé capable de produire une couleur en réagissant avec du peroxide d'hydrogène, des moyens (15) destinés à déterminer la couleur générée en mesurant la réflectance diffuse, des moyens (20) destinés à lire les résultats de mesure de la réflectance diffuse au moins à deux intervalles de temps préréglés après l'application du sang complet dans le coussin à échantillons (11), des moyens (20, 21) destinés à convertir la différence qui existe entre les résultats de mesure de la réflectance diffuse en une valeur de concentration de la glucose dans le sang complet, et des moyens (22) pour l'affichage de la valeur de la concentration de glucose ainsi obtenue.

FIG.1.

FIG.2.

REAGENT "STRIP"

FIG.3.